Europäisches Patentamt

European Patent Office

Office européen des brevets

(19)

(11) Publication number: **0 093 541**
A2

# (12) EUROPEAN PATENT APPLICATION

(21) Application number: 83302237.9

(22) Date of filing: 20.04.83

(51) Int. Cl.³: **A 61 K 7/06**
**C 07 D 213/89**

(30) Priority: 29.04.82 GB 8212524

(43) Date of publication of application:
09.11.83 Bulletin 83/45

(84) Designated Contracting States:
AT BE CH DE FR GB IT LI LU NL SE

(71) Applicant: BEECHAM GROUP PLC
Beecham House Great West Road
Brentford Middlesex(GB)

(72) Inventor: Davis, Walter Bryan
Beechcroft 25 Ralliwood Road
Ashtead Surrey(GB)

(72) Inventor: Fairhurst, Edgar
48 Warenne Road Fetcham
Leatherhead Surrey(GB)

(72) Inventor: Graham, June
45a The Broadway
Stoneleigh Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

(54) Antidandruff compositions.

(57) Zinc pyridine thione in particulate form wherein a major porportion of the particles each having a maximum diameter of $2\mu m$, and a maximum volume of $3 \times 10^{-17} m^3$ and a minimum surface area of $2 m^2/g$ and are in the form of hexagonal flat platelets or fragments thereof, is particularly efficacious in treating superficial disorders including dandruff and may be formulated in conventional presentations.

EP 0 093 541 A2

Croydon Printing Company Ltd.

## ANTIDANDRUFF COMPOSITIONS

The present invention relates to compositions containing zinc pyridine thione as antimicrobial agent, and to their use in treating or preventing disorders of the skin and scalp.

Zinc pyridine thione is a well known antimicrobial agent which is commonly incorporated into antidandruff compositions such as hair shampoos and conditioners. Whilst these generally achieve an antidandruff action the performance of such compositions is less than ideal because the detergents present in the compositions tend to reduce deposition of the ZPT on the hair and scalp, and that which is deposited is swiftly washed off during rinsing.

It has now been surprisingly found that, by selecting certain sizes of ZPT particles, formulations can be produced having improved antidandruff action. Moreover such ZPT formulations may be used in the treatment of various flaking and scaling skin disorders and other microbial conditions.

Accordingly, the present invention provides a hair or skin-care composition comprising zinc pyridine thione in association with a conventional carrier, characterised in that a major proportion of the zinc pyridine thione is in the form of particles each having a maximum diameter of 2 μm, and a maximum volume of 3 x $10^{-17}$ m$^3$ and a minimum surface area of 2 m$^2$/g, and being substantially in the form of hexagonal flat platelets or fragments thereof.

Compositions containing ZPT as described above have the advantages that the ZPT covers the hair and scalp and skin more efficiently, it is less readily removed during rinsing and has improved antidandruff activity.

Also, as the particles of ZPT are small and have a large surface area, they will be more rapidly solubilised in/on the scalp by either sebum or water, and hence be more effective than the standard larger particles during use.

Examples of the conditions which can be effectively alleviated are: dandruff, seborrheic dermatitis, tinea infections and "dry" flaking skin or winter xerosis.

Preferably at least 75% of the ZPT in the composition is in the form of particles as described above, more preferably at least 85% and most preferably at least 90%.

It will be appreciated that such particles may be produced by various procedures including crystallisation and that the particles will not all be in the form of perfect hexagonal platelets but many will be broken, agglomerated or incomplete.

Preferably the major proportion of ZPT particles have a maximum diameter of 1 μm.

Preferably the major proportion of the ZPT particles have a maximum volume of $4 \times 10^{-18}$ m$^3$.

Preferably the major proportion of the ZPT particles have a minimum surface area of 4 m$^2$/g.

The specified material may be presented in hair dressings, hair conditioners and shampoos. Other presentations of the composition include creams, ointments and lotions for treating facial, scalp and other superficial disorders.

Preferably compositions according to the present invention are presented as conventional hair shampoos or conditioners and include detergents such as sodium lauryl ether sulphate, sodium n-lauroyl sarcosinate, ammonium alkyl sulphide, and other conventional ingredients including thickeners, opacifiers, colourants, perfumes and preservatives.

Suitably the compositions according to the present invention are shampoos or conditioners, for instance as described in European Patent Application Nos 0 007 704 and 82300398 and U.K. Patent Application No. 8132141.

The size and shape of the the ZPT particles in a composition can be ascertained by conventional techniques such as Electron Microscopy, Coulter Counter or Centrifugal Particle Size Analysis.

Conveniently it may be ascertained by electron-microscopy.

Particles of ZPT for use in the compositions according to the present invention may be produced for instance by crystallisation of the zinc salt from solution of the more soluble sodium salt of pyridine thione. Alternatively they may be obtained from ZPT containing particles of all sizes by settling, optionally accelerated by centrifugation and selecting samples containing the appropriate distribution of particle sizes.

The present invention also provides a method for treating or preventing disorders of the skin and scalp which comprises applying a composition as hereinbefore defined to the hair, scalp and/or skin of the sufferer.

The invention will now be illustrated by the following Examples, which are not intended to limit the scope of the invention in any way.

0093541

Example 1

Production of ZPT fine platelets

1 Mole of zinc sulphate (161 g) was dissolved in distilled water containing 1% sodium lauryl ether sulphate. The solution was heated to 60°C.

2 Moles of sodium pyrithione (299 g) was dissolved in water and heated in a separated container, also to 60°C.

The two solutions were mixed and rapidly cooled. The crystals of zinc pyrithione formed were filtered with a glass micro filter, washed and dried. Their particle size was measured.

The crystals were then suspended in a suitable suspending agent (e.g. a surfactant/magnesium silicate system) and centrifuged.

Centrifugation time, speed and rotor angle are dependent on the viscosity and density of the solution and the density of zinc pyrithione particles (spherical shape is assumed), as governed by Stokes' Law:

$$D^2 = \frac{18\eta v}{(\rho s - \rho f)g}$$

$D$ = diameter of sphere  
$\eta$ = viscosity of fluid  
$v$ = terminal velocity  
$\rho s$ = density of sphere  
$\rho f$ = density of fluid  
$g$ = acceleration

After adequate centrifugation the supernatent was decanted, the crystals filtered and dried. Their particle size was remeasured, and if <2 µm they were stored in a dark container at 0°C.

Example 2

Formulations containing ZPT fine platelets

1) Shampoo                                                    % w/w
   Sodium lauryl ether sulphate                               55
     (27½% aqueous)
   Coconut monoethanolamide                                   4
   Ethylene Glycol Distearate                                 1
   Magnesium Aluminium Silicate                               1
   Zinc pyridine thione crystals                              2
     from Example 1
   Sodium chloride, perfume, colour                           qs
   Water                                             qs to 100

2) Cream Rinse
   Stearalkonium chloride                                     3
   Glycerol Monostearate                                      1.5
   Lanolin                                                    2
   Magnesium Aluminum Silicate                                0.8
   ZPT crystals from Example 1                                0.5
   Perfume, colour                                            qs
   Water                                                to 100

3) Hair dressing
   A water-in-oil emulsion is produced from
   the following ingredients:
   Mineral oil                                                32
   Beeswax                                                    2.5
   Perfume                                                    qs
   Calcium hydoxide solution (saturated)    qs to 100

4) <u>Facial and/or Body Cream</u>

| | |
|---|---:|
| Arlacel 186 (emulsifier) | 1.5 |
| Sorbitol | 7.0 |
| Ceresin Wax | 2.0 |
| Beeswax | 2.0 |
| Mineral oil | 5.0 |
| Petroleum jelly | 5.0 |
| Isopar L | 5.0 |
| Nipabutyl preservative (Butyl paraben) | 0.06 |
| Bronopol preservative (2-bromo-2-nitro-1,3-propanediol) | 0.02 |
| ZPT crystals from Example 1 | 0.10 |
| Water to | 100 |

4) <u>Facial and/or Body Cream</u>

## Biological Data

a) <u>Antidandruff trial</u>

A double blind clinical trial using human
subjects, and a subjective technique to evaluate
dandruff levels has shown smaller particle sized zinc
pyrithione to be more effective at alleviating
dandruff. After 6 weeks of treatment, volunteers in
the group receiving the base shampoo showed no change
in their dandruff level (8 volunteers). Subjects in
the group receiving a 1% zinc pyrithione shampoo with
an average particle size of 2.5 μm (high proportion <1
μm) showed a group mean percentage reduction in
dandruff levels of 60% (9 volunteers). Subjects in the
group receiving a 1% zinc pyrithione shampoo with an
average particle size of 7 μm (high proportion > 1 μm)
showed a group mean percentage reduction in dandruff
levels of only 40% (9 volunteers).

b) <u>Antimicrobial activity in vitro</u>

Two ZPT fractions were prepared in aqueous
suspension such that they differed in mean particle
size and aspect ratio. The fractions consisted of one
enriched in the small-particle ZPT hexagonal platelets
described above and another consisting of relatively
large-particle ZPT which had been depleted of the
small-particle material by repeated centrifugation and
washing. These fractions were assayed using the
commonly accepted and most reputable <u>in vitro</u> test of
anti-dandruff activity: antimicrobial activity against
<u>Pityosporum ovale</u> via agar diffusion. <u>Pityosporum
ovale</u> was grown on agar plates containing filter paper
discs or wells into which the test suspensions (5
replicates) were introduced. Plates were incubated at
37°C and zones of inhibition of yeast growth were determined after 48 hours.  At all concentrations of ZPT suspension tested in the range 0.03-0.61g%, small particle ZPT was found to yield larger zones of inhibition of yeast growth than did the large-particle material.

1.  A hair or skin-care composition comprising zinc pyridine thione in association with a conventional carrier, characterised in that a major proportion of the zinc pyridine thione is in the form of particles each having a maximum diameter of 2 µm, and a maximum volume of $3 \times 10^{-17}$ $m^3$ and a minimum surface area of 2 $m^2/g$, and being substantially in the form of hexagonal flat platelets or fragments thereof.

2.  A composition as claimed in claim 1 wherein at least 75% of the ZPT in the composition is in the form of particles as defined in claim 1.

3.  A composition as claimed in claim 1 or claim 2 wherein the major proportion of ZPT particles have a maximum diameter of 1 µm.

4.  A composition as claimed in any one of claims 1 to 3 wherein the major proportion of the ZPT particles have a maximum volume of $4 \times 10^{-18}$ $m^3$.

5.  A composition as claimed in any one of claims 1 to 4 wherein the major proportion of the ZPT particles have a minimum surface area of 4 $m^2/g$.

6.  A composition as claimed in any one of claims 1 to 5 presented as a hair dressing, hair conditioner, shampoo, cream, ointment or lotion for treating facial, scalp and other superficial disorders.

7.  A composition as claimed in claim 6 presented as a hair shampoo or conditioner and also comprising detergent selected from sodium lauryl ether sulphate, sodium n-lauroyl sarcosinate and ammonium alkyl sulphide.

8.  A process for producing a composition as claimed in any one of claims 1 to 7 which process comprises admixing the ZPT particles with the carrier.

9.  Zinc pyridine thione characterised in that it is in particulate form and a major proportion of the particles have a maximum diameter of 2$\mu$m and a maximum volume of $3 \times 10^{-17}m^3$ and a minimum surface area of $2m^2/g$ and are substantially in the form of hexagonal flat platelets or fragments thereof.

10. Zinc pyridine thione as claimed in claim 9 for use in treating the human or animal body.